(19) Europäisches Patentamt
European Patent Office
Office européen des brevets

(11) Veröffentlichungsnummer: **0 149 163**
A2

(12) **EUROPÄISCHE PATENTANMELDUNG**

(21) Anmeldenummer: **84115538.5**

(22) Anmeldetag: **15.12.84**

(51) Int. Cl.⁴: **A 61 K 31/70**
C 07 H 3/06

(30) Priorität: **27.12.83 DE 3347163**

(43) Veröffentlichungstag der Anmeldung:
**24.07.85 Patentblatt 85/30**

(84) Benannte Vertragsstaaten:
**AT BE CH DE FR GB IT LI LU NL SE**

(71) Anmelder: **BEHRINGWERKE Aktiengesellschaft**
**Postfach 1140**
**D-3550 Marburg 1(DE)**

(72) Erfinder: **Hermentin, Peter, Dr.**
**Barfüssertor 30**
**D-3550 Marburg(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) **Verwendung von Oligosacchariden zur Chemotherapie oder Chemoprophylaxe der Malaria.**

(57) Es wird beschrieben, daß eine Zucker-Kette, die ein Trimannosyl-Pentasaccharid der Formel 1

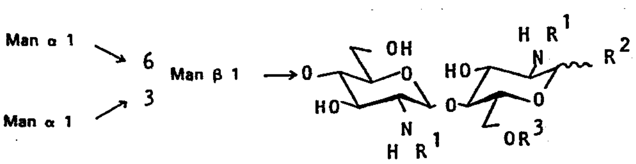

1

enthält,

worin $R^1$ Wasserstoff oder Acetyl (Ac) und $R^2$ $-OH$, $-NH_2$ oder $-NHNH_2$ oder $R^1$ Acetyle und $R^2$ $-$ NHAc oder $-NHNHAc$ und $R^3$ Wasserstoff oder α -L-Fucopyranosyl ist und die mittels Hydrazinolyse aus Glycoproteinen erhalten wird, zur chemotherapie oder Chemoprophylaxe der Malaria verwendet werden kann.

EP 0 149 163 A2

Verwendung von Oligosacchariden zur Chemotherapie oder Chemoprophylaxe der Malaria

Die Erfindung betrifft die Verwendung von aus Glycoproteinen isolierten Oligosacchariden oder Zucker-Ketten zur Chemotherapie oder Chemoprophylaxe der Malaria.

Die Entwicklung neuer Medikamente zur Chemotherapie und Chemoprophylaxe der Malaria ist wegen der zunehmenden Resistenzbildung der Erreger (Plasmodien) von steter und dringender Notwendigkeit.

Auch die Entwicklung eines Impfstoffes gegen die Malaria stellt nach wie vor ein unbewältigtes Problem höchster Priorität dar.

Es ist bekannt, daß der Befall von Humanerythrozyten durch P. falciparum-Merozoiten in vitro durch Zusatz bestimmter Zucker blockiert werden kann (M. Jungery, G. Pasvol, C. I. Newbold und D. J. Weatherall (1983), Proc. Natl. Acad. Sci. USA 80, 1018).

Nicht bekannt ist bislang, welche Zucker-Strukturen der Erythrozyten-Membran oder der Sialoglycoproteine beim Invasionsvorgang eine Rolle spielen.
Bekannt ist lediglich, daß über die Inhibitionswirkung der sechs auf dem Glycophorin A lokalisierten Monosaccharide widersprüchliche Ergebnisse vorliegen (M. Jungery, G. Pasvol, C. I. Newbold und D. J. Weatherall (1983), Proc. Natl. Acad. Sci. USA 80, 1018; R. J. Howard, J. D. Haynes, M. H. McGinniss und L. H. Miller (1982), Mol. Biochem. Parasit. 6, 303; M. M. Weiss, J. D. Oppenheim und J. P. Vanderberg (1981), Exp. Parasitol. 51, 400).

Bekannt ist weiterhin, daß auch Chitobiose eine invasionsinhibierende Wirkung besitzt (R. J. Howard, J. D. Haynes, M. H. McGinniss und L. H. Miller (1982), Mol. Biochem. Parasit. 6, 303).

Es ist auch bereits vorgeschlagen worden, N-Acetyl-D-glucosaminhaltige Mittel zur Behandlung der Malaria zu verwenden. (DE-A-32 20 426).

Keines der im Stand der Technik bekannten Produkte hat jedoch bisher in einem Mittel zur Verhütung oder Heilung von Malaria Verwendung gefunden.
Es bestand daher weiterhin die Aufgabe Stoffe aufzufinden, die zur Bekämpfung der Malaria brauchbar sind.

Überraschenderweise wurde nun gefunden, daß die mittels Hydrazinolyse aus Hühner-Ovomucoid oder -Ovalbumin gewonnenen, sogenannten "asparagin-verknüpften" Oligosaccharide, das Eindringen von *P. falciparum*-Merozoiten in Erythrozyten und/oder das Wachstum dieser Merozoiten in Erythrozyten hemmen.

Überraschenderweise gilt dies ebenso für mittels Hydrazinolyse gewonnene "asparagin-verknüpfte" Oligosaccharid-Gemische aus menschlicher Muttermilch oder $\beta_2$-Glycoprotein-I oder Glycoprotein-Gemische beispielsweise aus Eiern.

Es gilt weiterhin für die sauren (N-Acetylneuraminsäurehaltigen) und neutralen (N-Acetylneuraminsäure-freien) Oligosaccharid-Gemische, die durch Chromatographie beispielsweise an DEAE-Zellulose aus den mittels Hydrazinolyse aus Hühner-Ovomucoid oder menschlicher Muttermilch gewonnenen Oligosaccharid-Gemischen (Ovomucoid-Asn-OM und Muttermilch-Asn-OM) erhalten werden können.

Die Herstellung aller genannten Oligosaccharide erfolgt analog Beispiel 1.

Solche "asparagin-verknüpften" Oligosaccharide eignen sich zur Chemotherapie oder Chemoprophylaxe der Malaria, insbesondere der Malaria tropica.

Gegenstand der Erfindung ist daher die Verwendung von mittels Hydrazinolyse aus Glycoproteinen erhaltenen Zucker-Ketten, die ein Trimannosyl-Pentasaccharid der Formel 1

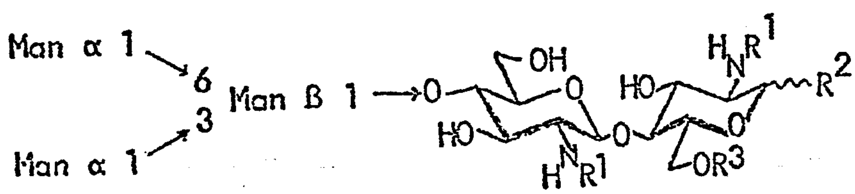

1

enthalten,

worin $R^1$ Wasserstoff oder Acetyl (Ac) und $R^2$ -OH, $-NH_2$ oder $-NHNH_2$ oder $R^1$ Acetyl und $R^2$ -NHAc oder -NHNHAc und $R^3$ Wasserstoff oder α-L-Fucopyranosyl ist, zur Chemotherapie oder Chemoprophylaxe der Malaria.

Solche Zucker-Ketten besitzen eine Verbindung der Formel 1 als Herzstück oder "Core"-Struktur und können Oligosaccharide vom sogenannten "High mannosetype" (Struktur 2), "Complex type" (Struktur 3) oder "Hybrid-type" (Struktur 4) (Abb. 1) sein, wobei an die jeweils freien Mannose-Reste weitere Zucker, wie zum Beispiel GlcNAc, Gal oder NeuAc, gebunden sein können.

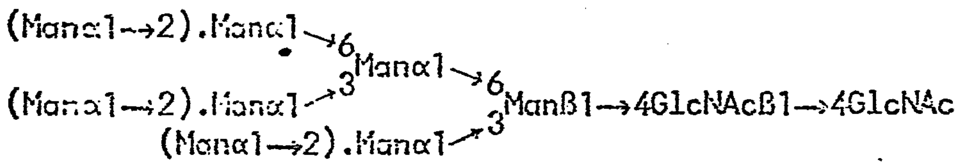

2

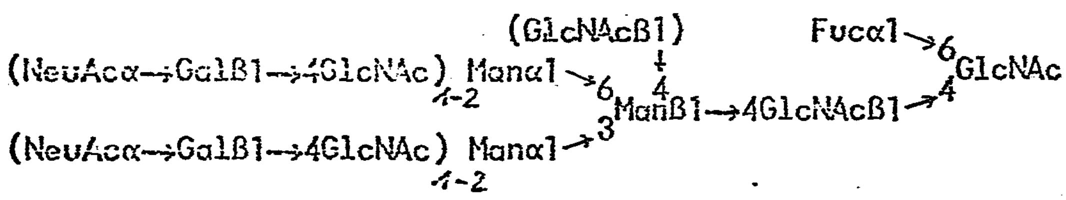

3

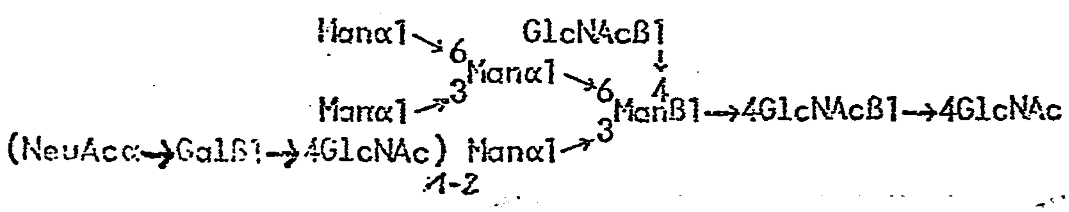

4

Diese Zucker-Ketten können ein Gemisch verschiedener, das Trimannosyl-Pentasaccharid der Formel 1 enthaltende Oligosaccharide oder ein einzelnes, gereinigtes solches Oligosaccharid sein.

Solche Zucker-Ketten können saure (N-Acetylneuraminsäure-haltige) oder neutrale (N-Acetylneuraminsäurefreie) Oligosaccharide oder Oligosaccharid-Gemische sein.

Die Zucker-Ketten werden aus den diesen Zucker-Typ enthaltenden Glycoproteinen in an sich bekannter Weise (S. Takasaki, T. Mizuochi und A. Kobata (1982), Methods Enzymol. 83, 263) durch Hydrazinolyse gewonnen, wobei Zucker-Ketten erhalten werden, die in ihrem einen Ende eine Chitobiose-Einheit ($R^3$=H) oder eine fucosylierte Chitobiose-Einheit ($R^3$= $\alpha$-L-Fucopyranosyl) enthalten.

Oligosaccharid-Gemische können gegebenenfalls in an sich bekannter Weise durch Gelfiltration, zum Beispiel über Biogel[R] P4, Ionenaustausch-Chromatographie, zum Beispiel an DEAE-Zellulose, high performance liquid chromatography (HPLC) oder durch Fraktionierung mit Hilfe von Lectinen, wie beispielsweise Lentil lectin, erythroagglutinierendes Phythemagglutinin (E-PHA), Ricinus communisagglutinin (RCA) oder Concanavalin A, gereinigt oder aufgetrennt werden, wobei die Lectine zweckmäßigerweise an einen festen Träger, wie zum Beispiel Sepharose 4 B oder Agarose gekoppelt sind.

Die Oligosaccharide können auch dadurch gewonnen werden, daß die den Zucker-Typ enthaltenden Glycoproteine vor der Hydrazinolyse-Reaktion in an sich bekannter Weise durch Glycosidasen, wie zum Beispiel Neuraminidase und/oder ß-Galactosidase vorbehandelt werden, wobei nach

der Hydrazinolyse ein einheitlicheres Oligosaccharid-Gemisch erhalten wird.

Die Oligosaccharide können auch dadurch vereinheitlicht werden, daß man im voranstehenden genannte Maßnahmen kombiniert. Die Glycosidasen können an einen festen Träger gekoppelt sein, was ihre Wiederentfernung aus der Oligosaccharid-Mischung durch einfache Zentrifugation erlaubt, oder werden auf einem anderen Wege, etwa durch Gelfiltration, wieder entfernt.

Geeignete Oligosaccharide können auch dadurch gewonnen werden, daß man die den Zucker-Typ enthaltenden Glycoproteine vor der Hydrazinolyse-Reaktion in an sich bekannter Weise (R. G. Spiro, Methods Enzymol. (1972), 28, 3-43) durch Enzyme, bevorzugt Pronase, in Glycopeptid-Fragmente aufspaltet und diese in an sich bekannter Weise durch Gelfiltration, zum Beispiel über Sephadex G 25 oder Biogel P4 oder Chromatographie über Dowex 50 auftrennt, gegebenenfalls durch Glycosidasen weiter abbaut und die Oligosaccharide schließlich durch Hydrazinolyse der lyophilisierten Glycopeptide gewinnt. Gegebenenfalls werden die durch Hydrazinolyse erhaltenen Produkte mit Acetanhydrid/Alkalibikarbonat acetyliert.

Den geeigneten Zuckertyp enthaltende Glycoproteine sind beispielsweise Ovomucoid, Ovalbumin, $\beta_2$-Glycoprotein-I, $\alpha_1$-saures Glycoprotein (Orosomucoid), Fetuin, Hühner-Ovotransferrin, Immunglobuline, Zymogene (zum Beispiel Prothrombin), Sekretorprotein der Muttermilch, Glycophorin A und andere Glycoproteine.

Diesen Zuckertyp enthaltende Glycoprotein-Gemische sind beispielsweise Hühnereiweiß, Hühnereigelb, Muttermilch

oder Kolostrum.

Die Isolierung der das Trimannosyl-Pentasaccharid der Formel 1 enthaltenden Zucker-Ketten erfolgt aus einem den Zuckertyp enthaltenden oben genannten Glycoprotein oder aus einem oben genannten lyophilisierten Glycopeptid-Fragment oder aus einer oben genannten lyophilisierten Glycoprotein-Mischung.

Die Isolierung der das Trimannosyl-Pentasaccharid der Formel 1 enthaltenden Zuckerketten erfolgt bevorzugt aus Ovomucoid, Ovalbumin oder Muttermilch, besonders bevorzugt aus Ovomucoid oder lyophilisierter Muttermilch und im besonderen aus Ovomucoid.

Die Isolierung dieser Zuckerketten erfolgt aber auch bevorzugt aus Hühnereiern, aus Hühnereiweiß, aus Hühnereigelb oder Kolostrum, wobei das Proteingemisch lyophilisiert sein muß.

Die Zuckerketten können auch bevorzugt aus Trockenmilch-Pulver und Trockenei-Pulver isoliert werden.

Die nachfolgenden Beispiele sollen die Erfindung erläutern:

Beispiel 1:

Gewinnung "asparagin-verknüpfter" Oligosaccharide durch Hydrazinolyse von Glycoproteinen, Glycopeptid-Fragmenten oder Glycoprotein-Mixturen

Ovomucoid (6 g) wurde in an sich bekannter Weise (S. Takasaki, T. Mizuochi und A. Kobata (1982), Meth. Enzymol. 83, 263) in wasserfreiem Hydrazin (60 ml) 8 Std., auf 100°C erhitzt. Die Reaktionsmischung wurde nachfolgend im Wasserstrahlvakuum unter Vorlage von konzentrierter Schwefelsäure am Rotationsverdampfer bei Raumtemperatur eingeengt, restliches Hydrazin durch wiederholtes Einengen in Gegenwart von Toluol und nachfolgende Trocknung im Ölpumpenvakuum entfernt, das verbliebene Produkt in einer gesättigten wäßrigen Lösung von Natriumhydrogencarbonat (6 ml) aufgenommen, freie Aminogruppen durch Zusatz von Acetanhydrid (1,2 ml) N-acetyliert, die Lösung mittels Dowex 50 W x 2 ($H^+$ form) entsalzt und filtriert, über eine Biogel P4-Säule in Ethanol/Wasser (1/9) und gegebenenfalls über eine der oben beschriebenen Methoden weiter gereinigt, und die Kohlenhydrate in an sich bekannter Weise detektiert, z. B. nach der Phenol-Schwefelsäure-Methode (M. Dubois, K. A. Gilles, J. K. Hamilton, P. A. Rebers und F. Smith (1956), Anal. Chem. 28, 350) oder durch Entwicklung von Kieselgel-Dünnschichtchromatogrammen mit Schwefelsäure bei ca. 150°C vermessen, und nach geeigneter Fraktionierung, etwa wie oben erwähnt, lyophilisiert.
Derartig isolierte Oligosaccharide oder Oligosaccharid-Mixturen der Formel 1 verhindern die Vermehrung von Malaria-Parasiten im Blut, was insbesondere im in vitro-Testsystem aufgezeigt werden kann.
Analog können die anderen vorstehend genannten Glycoproteine, Glycopeptid-Fragmente oder Glycoprotein-Mixturen behandelt werden.

Beispiel 2:
Verwendung von durch Hydrazinolyse von Glycoproteinen gewonnenen "asparagin-verknüpften" Oligosacchariden mit

Core-Struktur 1 zur Chemotherapie oder Chemoprophylaxe der Malaria.

Zur Chemotherapie oder Chemoprophylaxe der Malaria wird die gemäß vorstehendem Abschnitt gewonnene Oligosaccharid-Mixtur, die gegebenenfalls wie vorn beschrieben in an sich bekannter Weise weiter gereinigt oder aufgetrennt werden kann, oral oder parenteral, ein- oder mehrmals, gegebenenfalls in Verbindung mit einem humanverträglichen Hilfs- und/oder Zusatzstoff, in einer wirksamen Menge von zum Beispiel 0,1 mg bis 10 mg Wirkstoff je kg Körpergewicht verabreicht.

Patentansprüche

1. Verwendung von einer mittels Hydrazinolyse aus einem Glycoprotein erhaltenen Zucker-Kette, die ein Trimannosyl-Pentasaccharid der Formel 1

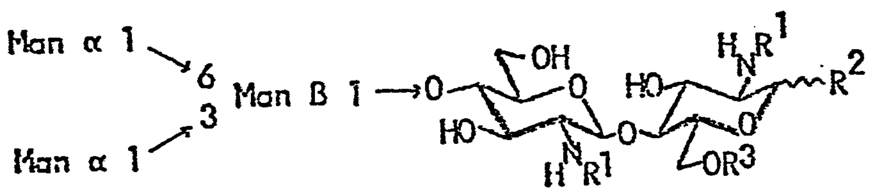

1

enthält,

worin $R^1$ Wasserstoff oder Acetyl (Ac) und $R^2$ -OH, $-NH_2$ oder $-NHNH_2$ oder $R^1$ Acetyl und $R^2$ - NHAc oder -NHNHAc und $R^3$ Wasserstoff oder α -L-Fucopyranosyl ist, zur Chemotherapie oder Chemoprophylaxe der Malaria.

2. Verwendung einer Verbindung nach Anspruch 1, dadurch gekennzeichnet, daß diese Verbindung aus Hühner-Ovomucoid, Hühner-Ovalbumin, menschlicher Muttermilch, $\beta_2$-Glycoprotein-I, $\alpha_1$-saurem Glycoprotein, Fetuin oder einem Glycoprotein-Gemisch aus Eiern erhalten wurde.

3. Verfahren zur Herstellung eines Mittels zur Chemotherapie oder Chemoprophylaxe der Malaria, dadurch gekennzeichnet, daß man ein Glycoprotein, das eine "asparagin-verknüpfte" Zuckerkette enthält, mit Hydrazin behandelt, eine Verbindung der Formel I in Anspruch 1, worin $R^1$, $R^2$ und $R^3$ die in Anspruch 1 angegebene Bedeutung haben, gewinnt und in eine geeignete Darreichungsform bringt.